# EUROPEAN PATENT SPECIFICATION

(11) **EP 1 677 627 B1**
(45) Date of publication and mention of the grant of the patent: **18.03.2009**
(21) Application number: 04762316.0
(22) Date of filing: 08.10.2004
(51) Int. Cl.: A23L 1/30, A61K 31/728, A61P 19/10

(54) **DIETETIC PREPARATION WITH HYALURONATE FOR TREATMENT OF OSTEOPOROSIS**
HYALURONHALTIGES DIÄTPRODUKT FÜR DIE BEHANDLUNG VON OSTEOPOROSE
PREPARATION DIETETIQUE CONTENANT UN HYALURONATE POUR LE TRAITEMENT DE L'OSTEOPOROSE

(30) Priority: 08.10.2003 CZ 200314679 U
(43) Date of publication of application: 12.07.2006
(73) Proprietor: CPN Spol. S R.O., 561 02 Dolni Dobrouc (CZ)
(72) Inventor: VELEBNY, Vladimir, 562 02 Usti nad Orlici (CZ); STANCIKOVA , Maria, 921 01 Piestany (SK); SVIK, Karol, 922 31 Sokolovce (SK); ISTOK, Richard, 921 01 Piestany (SK)
(74) Representative: Kania, Frantisek
(86) International application number: PCT/CZ2004/000064
(87) International publication number: WO 2005/032276

(56) References cited:
- WO-A1-97/33592
- WO-A1-20/04071543
- WO-A2-03/099992
- US-A1- 2003 050 277
- US-B1- 6 391 864
- US-B1- 6 495 148
- STANCÍKOVÁ M ET AL: "The effects of hyaluronan on bone resorption and bone mineral density in a rat model of estrogen deficiency-induced osteopenia" INTERNATIONAL JOURNAL OF TISSUE REACTIONS 2004 SWITZERLAND, vol. 26, no. 1-2, 2004, pages 9-16, XP008042023 ISSN: 0250-0868
- DATABASE WPI Section Ch, Week 200435 Derwent Publications Ltd., London, GB; Class B04, AN 2004-369103 XP002314986 & JP 2004 137183 A (KOTOBUKI SEIYAKU CO LTD) 13 May 2004 (2004-05-13)
- NAKAMURA KIMIHIDE ET AL: "High, but not low, molecular weight hyaluronan prevents T-cell-mediated liver injury by reducing proinflammatory cytokines in mice" JOURNAL OF GASTROENTEROLOGY, vol. 39, no. 4, April 2004 (2004-04), pages 346-354, XP008042013 ISSN: 0944-1174
- DATABASE WPI Section Ch, Week 200375 Derwent Publications Ltd., London, GB; Class B04, AN 2003-793480 XP002314988 & JP 2003 212781 A (MARUZEN SEIYAKU KK) 30 July 2003 (2003-07-30)

## Description

### Field of the invention

The present invention relates to a preparation based on sodium hyaluronate, and possibly on another pharmacologically acceptable salt of hyaluronic acid, formulated into tablets, chewing tablets, jelly capsules, an instant drink, a chewing gum, syrup, a bar or a candy acting preventively against osteoporosis and, in case of a diagnosis of the disease, being able to suppress its development and to contribute to therapy.

### Background of the invention

Osteoporosis represents a serious and widely spread problem for the elderly generation, especially for women after menopause. Statistics show that more than 50 % of women and more that 14% of men over 50 years suffer from some manifestation of osteoporosis. The most serious manifestation thereof with detrimental impact upon the patients as well as upon the health care system is the fact that osteoporosis increases the fragility of bones. Fractures, in particular fractures of the femural collum, lead to hospitalization as a considerable complication, especially for the group of very old people.

The prevention of osteoporosis, is very problematic and not efficient enough. In effect, the only method for preventing osteoporosis is sufficient supplementation of calcium in the form of tablets or other preparations, especially for the seniors. This method has also its limitations, not every form of calcium preparation and not every form of supplied calcium can be fully utilized. In spite of the declarations of manufacturers no protective effect against osteoporosis was proved for dietary preparations based on hydrolysates of collagen protein.

A number of drugs are used to treat osteoporosis, but as far as efficient, these medicaments are burdened with a range of side effects. Three most frequently used groups can be offered as example, namely medicaments for hormone replacement therapy, bisphosphonates and calcitonin.

As to drugs used for substitution hormone therapy, substantial venous thromboembolitic complications and increased frequency of the breast gland carcinoma and of the endometrium were ascertained. For women who took this therapy more than 5 years, the risk of breast gland cancer, risk of cardiovascular diseases and risk of cerebrovascular accident increased significantly. This therapy can also cause irregular bleeding, liver function failure, gallbladder diseases and hypertension. The risk of endometrium cancer grows when taking estrogens without progestin/progesterone or without sufficient doses thereof.

When ingesting medicaments from the group of bisphosphonates, symptoms of erosive esophagitis or gastritis, reactivation of gastric ulcers, nausea, vomiting, heart throbbing, muscle pain, joint pain and bone pain can occur.

Administering of calcitonin causes the least side-effects. Absolute contraindication to administering calcitonin is hypersensitivity to calcitonin. Taking calcitonin in the form of a nasal spray can cause irritation of the nasal mucosa and it can also lead to small epistaxis or nasal ulceration.

The international publication WO 97/25 051 describes the administering of hyaluronate in the form of preparation for the prevention of re-stenosis after balloon angioplasty and as prevention of infarction. Also the international publication WO 97/40 841 shows administering thereof as an anti-neoplastic substance. According to a Japanese patent (JP 10 7550) it is recommended as a substance suitable for the prevention and treatment of pain in the throat and of nasopharyngitis.

### Subject-matter of the invention

The aim of the invention is to create a preparation enabling preventative action against the development of osteoporosis, and in case the manifestation of the disease has started, to suppress further development thereof. The preparation should not have any undesirable side effects. Another characteristics of the preparation is an easy form of administration that is pleasant for the patient.

The disadvantages stated in the background art of the invention and the aims laid out above are solved by the preparation for prevention and treatment of osteoporosis according to the invention. The subject matter of the invention is a preparation containing at least one physiologically acceptable salt of hyaluronic acid having molecular weight within the range from 500 000 to 4 000 000 g/mol and being in a form that is suitable for oral administration. A physiologically acceptable salt is preferably selected from the group comprising sodium salt, potassium salt, zinc salt, magnesium salt or calcium salt, More preferably the preparation according to the invention contains calcium salt of hyaluronic acid.

The preparation according to the invention preferably contains a physiologically acceptable inorganic salt in an amount ensuring that the daily dose of every single form of administration contains from 5 to 300 mg of a physiologically acceptable salt of hyaluronic acid.

The preparation according to the invention is either in a solid dosage form, such as tablets, chewing tablets, hard capsules, chewing gum, bars or candies, or in a liquid dosage form, such as an instant drink or syrup, where, in these administration forms, it is present either alone or in a blend with calcium salts or other victuals, such as preferably coconut flour, dried mill inverted syrup, vitamin premix, chewing substance, de-mineralized water, fruit flavour. A preparation according to the invention in liquid form, such as an instant drink or syrup, further contains potassium sorbate as a preservative.

The above mentioned dosage forms are pleasant for the patient and at the same time they do not require any special devices and are applicable anywhere in the field.

It is obvious from our not yet published experimental results that sodium hyaluronate or calcium hyaluronate are able to suppress symptoms of osteoporosis in female rats after surgically removed ovaries, if hyaluronate is administered orally.

### Examples of the invention

### Example 1

600 g of inverted syrup was mixed with 300 g of de-mineralized water and 3 g of sodium hyaluronate having molecular weight of 1 000 000 g/mol. The mixture was flavoured by apple flavour and preserved with potassium sorbate. Final syrup was used as a nutraceutical, the daily dosage was 30 ml.

### Example 2

30 g of coconut flour was mixed with 30 g of dried milk, 0,5 g of dolomite, 30 g of inverted syrup and 0,05 g of sodium hyaluronate having molecular weight 2 500 000 g/mol. The mixture was flavoured by vanilla flavour and formed into the shape of a bar. The product was used as a nutraceutical containing the daily dosage of hyaluronate.

### Example 3

100 g of tablet-forming substance for chewing tablets was mixed with 5 g of calcium hyaluronate and 5 g of sodium hyaluronate having molecular weight 1 500 000 g/mol, flavoured by sour cherry flavour and formed into a shape of cylindrical tablets having the weight of 2 g. The product was used as a nutraceutical with the daily dosage of sodium hyaluronate in one tablet.

### Example 4

An experiment was carried out on ovarectomised female rats in which an experimental osteoporosis was induced. It was shown that oral administering of sodium hyaluronate having molecular weight over 500 000 g/mol, and in a dosage in the range of 150 to 4500 pg/kg, leads to normalisation of bone density which was reduced in the laboratory model used. Also the excretion of the metabolite of bone matrix breakdown into urine of the laboratory animals returned to normal as a sign that the bone reduction was stopped.

## Claims

1. The use of a physiologically acceptable salt of hyaluronan having the molecular weight from 500 000 to 4 000 000 g/mol for manufacturing a preparation for prevention and treatment of osteoporosis by oral administration.

2. The use of a physiologically acceptable salt of hyaluronan according to claim 1 **characterized in that** hyaluronan is in the forms of physiologically acceptable inorganic salt selected from the group comprising sodium salt, potassium salt, zinc salt, magnesium salt or calcium salt.

3. The use of a physiologically acceptable salt of hyaluronan according to claims 1 to 2 **characterized in that** the daily dose of the preparation contains 5 to 300 mg of a physiologically acceptable salt of hyaluronan for prevention and treatment of osteoporosis by oral administration.

4. The use of a physiologically acceptable salt of hyaluronan according to claims 1 to 3 **characterized in that** the preparation is in solid forms such as tablets, bars, chewing tablets, hard capsules.

5. The use of a physiologically acceptable salt of hyaluronan according to claims 1 to 3 **characterized in that** the preparation is in liquid forms such as syrups and instant drinks.

## Patentansprüche

1. Verwendung von einem physiologisch verträglichen Salz von Hyaluronan, dessen Molekulargewicht 500 000 bis 4 000 000 g/mol beträgt, zur Herstellung einer Zubereitung zur Vorbeugung und Behandlung von Osteoporose durch orale Verabreichung.

2. Verwendung von einem physiologisch verträglichen Salz von Hyaluronan nach Anspruch 1, **dadurch gekennzeichnet, dass** Hyaluronan in der Form von einem physiologisch verträglichen inorganischen Salz ist, das aus der Natronsalz, Kaliumsalz, Zink-Salz, Magnesiumsalz oder Kalziumsalz umfassenden Gruppe gewählt ist.

3. Verwendung von einem physiologisch verträglichen Salz von Hyaluronan nach Anspruch 1 bis 2, **dadurch gekennzeichnet, dass** die Tagesdosis der Zubereitung 5 bis 300 mg eines physiologisch verträglichen Salzes von Hyaluronan zur Vorbeugung und Behandlung einer Osteoporose durch orale Verabreichung enthält.

4. Verwendung von einem physiologisch verträglichen Salz von Hyaluronan nach Anspruch 1 bis 3, **dadurch gekennzeichnet, dass** die Zubereitung in festen Formen ist, wie zum Beispiel Tabletten, Stäbchen, Kautabletten, harte Kapseln.

5. Verwendung von einem physiologisch verträglichen Salz von Hyaluronan nach Anspruch 1 bis 3, **dadurch gekennzeichnet, dass** die Zubereitung in flüssigen Formen ist, wie zum Beispiel Sirupe oder Instant-Getränke.

## Revendications

1. Utilisation d'un sel pharmaceutiquement acceptable d'un hyaluronane ayant le poids moléculaire de 500 000 à 4 000 000 g/mol pour la préparation d'une préparation déstinée à la prévention et traitement de l'ostéoporose par l'administration orale.

2. Utilisation d'un sel pharmaceutiquement acceptable d'un hyaluronane selon la revendication 1, **caractérisée en ce que** l'hyaluronane est sous forme d'un sel minéral pharmaceutiquement acceptable choisi du groupe contenant un sel de sodium, un sel de potassium, un sel de zinc, un sel de magnésium ou un sel de calcium.

3. Utilisation d'un sel pharmaceutiquement acceptable d'un hyaluronane selon la revendication 1 ou 2, **caractérisée en ce que** la dose journalière de la préparation contient de 5 à 300 mg d'un sel pharmaceutiquement acceptable d'un hyaluronane pour la prévention et traitement de l'ostéoporose par l'administration orale.

4. Utilisation d'un sel pharmaceutiquement acceptable d'un hyaluronane selon les revendications 1 à 3, **caractérisée en ce que** la préparation est sous forme des comprimés, barres, comprimés à mâcher, capsules dures.

5. Utilisation d'un sel pharmaceutiquement acceptable d'un hyaluronane selon les revendications 1 à 3, **caractérisée en ce que** la préparation est sous forme liquide, par exemple des sirops ou des boissons instantanées.
